(19) Europäisches Patentamt
European Patent Office
Office européen des brevets

(11) Publication number: **0 543 555 A1**

(12) **EUROPEAN PATENT APPLICATION**

(21) Application number: **92310277.6**

(51) Int. Cl.$^5$: **A61K 31/665**

(22) Date of filing: **11.11.92**

(30) Priority: **18.11.91 EP 91310608**

(43) Date of publication of application:
**26.05.93 Bulletin 93/21**

(84) Designated Contracting States:
**AT BE CH DE DK ES FR GB GR IE IT LI LU MC NL PT SE**

(71) Applicant: **MERRELL DOW PHARMACEUTICALS INC.**
**2110 East Galbraith Road**
**Cincinnati Ohio 45215−6300(US)**

(72) Inventor: **Cardin, Alan D.**
**9903 Hunters Run Lane**
**Cincinnati, Ohio 45242(US)**
Inventor: **Tyms, A. Stanley**
**Flat 8, 59 Bassett Road**
**North Kensington, London W10 6JR(GB)**

(74) Representative: **Perry, Robert Edward**
**GILL JENNINGS & EVERY, Broadgate House,**
**7 Eldon Street**
**London EC2M 7LH (GB)**

(54) **Phosphorylated flavanoids in the treatment of viral diseases.**

(57) Certain phosphorylated flavanoids are disclosed to be effective in treating retroviral infections including HIV infections as well as infections of herpes simplex virus and cytomegalovirus.

EP 0 543 555 A1

Rank Xerox (UK) Business Services
(3. 10/3.6/3.3. 1)

This invention relates to the use of certain phosphorylated flavanoids in the treatment of retroviral infections including HIV infections, as well as the treatment of cytomegalovirus (CMV) and Herpes Simples Virus (HSV) Types I and II.

## BACKGROUND OF THE INVENTION

A great deal of research is currently underway to develop treatments and cures for viral infections in humans and in animals. Notably the incidence of AIDS and ARC in humans is increasing at an alarming rate. The five year survival rate for those with AIDS is dispiriting and AIDS patients, whose immune systems have been seriously impaired by the infection, suffer from numerous opportunistic infections including Kaposi's sarcoma and *Pneumocystis carninii* pneumonia. No cure is known and current treatments are largely without adequate proof of efficacy and have numerous untoward side effects. Fear of the disease has resulted in social ostracism of and discrimination against those having or suspected of having the disease.

Retroviruses are a class of ribonucleic acid (RNA) viruses that replicate by using reverse transcriptase to form a strand of complementary DNA (cDNA) from which a double stranded, proviral DNA is produced. This proviral DNA is then randomly incorporated into the chromasomal DNA of the host cell making possible viral replication by later translation of the integrated DNA containing the viral genome.

Many of the known retroviruses are oncogenic or tumor causing. Indeed the first two human retroviruses discovered, denoted human T-cell leukemia virus I and II or HTLV-I and II, were found to cause rare leukemias in humans after infection of T-lymphocytes. The third such human virus to be discovered, HTLV-III, now referred to as HIV, was found to cause cell death after infection of T-lymphocytes and has been identified as the causative agent of acquired immune deficiency syndrome (AIDS) and AIDS related complex (ARC).

Retroviruses have, in addition to the usual viral capsid, an outer envelope of lipid and glycoprotein. The lipid of the retroviral membrane is probably derived directly from the membrane of a previously infected host cell, however, the glycoprotein of the viral membrane is unique to the virus itself and is coded for by the viral genome. Infection of a host cell by a retrovirus initially relies on the interaction of various receptors on the host cell surface, in particular CD4, with the glycoproteins of the envelope of the virus. Subsequently the virus and cell membranes fuse and the virion contents are released into the host cell cytoplasm. The glycoprotein envelope of the retroviruses plays an important role in both the initial interaction of the virion and the host cell and in the later fusion of the viral envelope and with host cell membranes.

Applicants have discovered that a class of phosphorylated flavanoids are active against HIV. Herpes Simplex Virus (HSV) types I and II, as well as cytomegalovirus (CMV), have functionally related glycoprotein coatings and their viral infectivity can also be diminished or eliminated by the use of the phosphorylated flavanoids of this invention.

## SUMMARY OF THE INVENTION

The present invention is directed to the antiretroviral, anti-HSV and anti-CMV activity of certain phosphorylated flavanoids. More particularly, it is directed to antiviral activity of the phosphorylated flavanoids having the following formula:

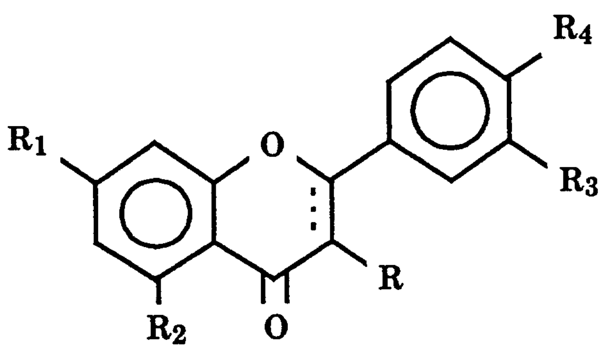

1

wherein R and $R_1$ are each independently selected from the group consisting of hydrogen, hydroxy, methoxy, and phosphonoxy as well as a saccharide linked via a hydroxy group selected from glucose,

ribose, fructose, galactose, mannose and disaccharides composed of these saccharides;

$R_2$, $R_3$, and $R_4$ are each independently selected from the group consisting of hydrogen, hydroxy, methoxy, and phosphonoxy;

and wherein the dotted line indicates the optional presence of a double bond;

with the proviso that at least one of R, $R_1$, $R_2$, $R_3$, and $R_4$ must be a phosphonoxy;

or a pharmaceutically acceptable acid addition salt thereof.

## DETAILED DESCRIPTION OF THE INVENTION

The chemical nature of the compounds of Formula 1 includes a natural propensity to polymerize. The precise structural nature of the polymer is not known and involves various modes of chemical linkages between the monomeric units. Nevertheless, the polymeric substances also exhibit antiviral activity and are useful either as mixtures of polymeric substances or as substantially purified substances of distinct polymer number whether a mixture of polymers resulting from natural or induced polymerization or as substantially pure substances of distinct polymer number resulting from controlled polymerization or by fractionation of a mixture. Fractionation can be accomplished by a variety of techniques including chromatography.

The expression "oligomers and fractions of oligomers" is intended to apply to dimers, trimers, tetramers and higher polymeric derivatives of the Formula 1 compounds either as a mixture of substances of narrow polymer numbers or as a substance of substantially one polymer number.

The expression "a pharmaceutically acceptable acid addition salt" is intended to apply to any non-toxic organic or inorganic acid addition salt of the base compounds. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono, di, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. These salts and the base compounds can exist in either a hydrated or a substantially anhydrous form. The acid salts are prepared by standard techniques such as by dissolving the free base in aqueous or aqueous-alcohol solution or other suitable solvents containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic organic solvents and which in comparison to their free base forms, demonstrate higher melting points and an increased solubility.

Preferred compounds of the present invention are those lacking the optional double bond. Also preferred are those compounds of formula 1 wherein R and $R_{1-4}$ are each either a hydrogen, hydroxy, methoxy, or phosphonoxy group. More preferred are those compounds of formula 1 wherein R is a hydrogen and those wherein $R_4$ is a methoxy. Even more preferred are those compounds of formula 1 wherein R is a hydrogen, $R_4$ is a methoxy, and $R_{1-3}$ are each a hydroxy or phosphonoxy group.

The phosphorylated flavanoids of the present invention are generally known and can be prepared by the reaction of phosphoric acid on the corresponding hydroxy substituted flavanoid by methods well known in the art.

The ability of the phosphorylated flavanoids of this invention to act as antiviral agents can be demonstrated by their ability to reduce syncytia formation and p24 antigen production in HIV infected human T-cells, JM and by using standard plaque reduction assays for herpes viruses (See examples 4 - 7).

The phosphorylated flavanoids of this invention can be used to treat a number of diseases and conditions known to be caused by viruses including those diseases and conditions caused by the retroviruses murine leukemia virus, feline leukemia virus, avian sarcoma virus, human immunodeficiency virus (HIV), HTLV-I, and HTLV-II, as well as cytomegalovirus (CMV) and herpes simplex virus (HSV) types I and II. Those experienced in this field are readily aware of the circumstances requiring antiviral therapy. Applicants consider the use of the phosphorylated flavanoids of this invention to treat HIV infections in humans to be of most importance. The term "patient" used herein is taken to mean mammals such as primates, including humans, sheep, horses, cattle, pigs, dogs, cats, rats and mice. The phosphorylated flavanoids can be used to prevent syncytium formation in cells infected with HIV-1, or other related viruses having related surface glycoprotein as well the herpes simplex viruses (HSV) I and II glycoproteins and the human cytomegaloviruses (CMV) glycoproteins. The phosphorylated flavanoids can

be used to treat AIDS and ARC and other diseases caused by the retrovirus HIV or other related viruses having gp120 surface glycoprotein as well as diseases caused by the herpes simplex viruses (HSV) I and II and cytomegalovirus (CMV). The amount of a phosphorylated flavanoid of formula 1 which is needed to prevent syncytium formation in HIV, HSV or CMV infected cells can be any effective amount. Experimentally, applicants have determined that phosphorylated flavanoids when employed at a concentration of 10 µg/ml resulted in complete inhibition of syncytium formation as well as a reduction in the presence of p24 antigen, an indicator of HIV viral replication, to below 3.0 x $10^2$ pg/ml.

The amount of the phosphorylated flavanoids of formula 1 to be administered can vary widely according to the particular dosage unit employed, the period of treatment, the age and sex of the patient treated, the nature and extent of the disorder treated, and the particular phosphorylated flavanoid selected. Moreover the phosphorylated flavanoids can be used in conjunction with other agents known to be useful in the treatment of retroviral diseases and glycoprotein coated viral diseases and agents known to be useful to treat the symptoms of and complications associated with diseases and conditions caused by retroviruses and viruses having glycoprotein coats. The anti-retrovirally effective amount of a phosphorylated flavanoid derivative of formula 1 to be administered will generally range from about 15 mg/kg to 500 mg/kg. A unit dosage may contain from 25 to 500 mg of the phosphorylated flavanoid derivative, and can be taken one or more times per day. The phosphorylated flavanoid derivative can be administered with a pharmaceutical carrier using conventional dosage unit forms either orally or parenterally.

The preferred route of administration is oral administration. For oral administration the phosphorylated flavanoids can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The phosphorylated flavanoids of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethylene-glycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutical adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the phosphorylated flavanoid derivative of formula 1 in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can

be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

EXAMPLE 1

Tablets are prepared each having the composition:

| | |
|---|---|
| Formula 1 phosphorylated flavanoid | 250 mg |
| starch | 40 mg |
| talc | 10 mg |
| magnesium stearate | 10 mg |

EXAMPLE 2

Capsules are prepared each having the composition:

| | |
|---|---|
| Formula 1 phosphorylated flavanoid | 400 mg |
| talc | 40 mg |
| sodium carboxymethylcellulose | 40 mg |
| starch | 120 mg |

EXAMPLE 3

Injectable dosages forms are prepared each having the composition:

| | |
|---|---|
| Formula 1 phosphorylated flavanoid | 0.500 g |
| polyoxyethylene sorbitan monooleate | 2.000 g |
| sodium chloride | 0.128 g |
| water for injection qs ad | 20.000 ml |

EXAMPLE 4

Anti-HIV activity of Phosphorylated Hesperidin

JM cells were infected with HIV-1 for 2 hours at room temperature. The cells were then washed and distributed into duplicate wells containing different concentrations of the test compound and incubated at 37°C. On days 3 and 4 the cells were observed and the syncytia counted and on day 4 the supernatant fluid was also harvested for detection of p24 viral core antigen using the Abbott EIA.

| Compound | Conc | Mean Syncytial Counts | | | | p24 (pg/ml) |
|---|---|---|---|---|---|---|
| | | Day 3 | % | Day 4 | % | |
| Virus Control (VC) | – | 57 | 100% | 92 | 100% | 1.5 x $10^5$ |
| Phosphorylated Hesperidin | 100 µg/ml | 0 | 0% | 5 | 5% | 1.5 x $10^4$ |
| | 30 | 9 | 16% | 45 | 48% | > 2.8 x $10^4$ |
| | 10 | 51 | 89% | As VC | 100% | > 2.8 x $10^5$ |
| | 3 | As VC | 100% | " " | 100% | " |
| | 1 | " " | 100% | " " | 100% | " |
| | 0.3 | " " | 100% | " " | 100% | " |
| | 0.1 | " " | 100% | " " | 100% | " |
| ddC | 100µM | Toxic | | | | |
| | 30 | Toxic | | | | |
| | 10 | 0 | 0% | 0 | 0% | 132 |
| | 3 | 0 | 0% | 0 | 0% | 98 |
| | 1 | 0 | 0% | 0 | 0% | 199 |
| | 0.3 | 0 | 0% | 6 | 7% | 341 |
| | 0.1 | 21 | 37% | 31 | 37% | > 2.3 x $10^3$ |

EXAMPLE 5

Anti – HIV activity of Fractionated Phosphorylated Hesperidin

Compound: HPLC Fractionated Phosphorylated Hesperidin
MDL 4943
29 Fractions

Experimental on the Fractionation Process

200 ml of 10 mg/ml phosphorylated hesperidin in 5% aqueous acetonitrile containing 0.1% trifluoroacetic acid (TFA) (solvent A) was injected onto a 4.6 x 15 cm Lichrospher® C18 reverse phase column (5 µm particle size) equilibrated in solvent A. The column was eluted with a linear gradient of 0% solvent A to 100% solvent B (60% aqueous acetonitrile, 0.1% TFA) over 30 minutes at a flow rate of 1 ml/minute. In these experiments successive 1 ml fractions were collected in Fraction No. 1 corresponded to the first sample collected between 1.0 – 2.0 minutes after initiation of the gradient program. The solvent was then removed by vacuum centrifugation and then reconstituted in RPMI media for antiviral testing as described.

1.5 ml of RPMI were added to each tube to redissolve the fractionated material. The fractions were then assayed for anti – HIV activity by adding 1 ml of each fraction of phosphorylated hesperidin to a well of a tissue culture plate, followed by 100 syncytial forming units of HIV and 1 x $10^5$ JM cells. The plates were incubated at 37° C for 2 days and then the syncytia scored as per cent of control (no compound added).
Results: 0 = 0%, + = 1 – 50%; + + = 50 – 100%; + + + = >100%

| Fraction No. | Syncytia |
|---|---|
| 1 – 13 | + + + |
| 14 | + /0 |
| 15 | + /0 |
| 16 | + /0 |
| 17 | 0 |
| 18 | 0 |
| 19 | + + |
| 20 – 29 | + + + |

EXAMPLE 6

Anti – CMV Activity of Phosphorylated Hesperidin

Experiments were carried out whereby phosphorylated hesperidin was present at the following times of the virus (AD169 strain, MRC 5 cell line) growth cycle.

1. During adsorption and after
2. During adsorption, then not
3. Only added after adsorption

A virus control with no drug was present on each plate.

| First trial results: | | | | | | |
|---|---|---|---|---|---|---|
| Compound | Concentration | No. of Plaques | | | Average | % |
| Virus Control | – | 240 | 237 | 249 | 242 | 100% |
| Phosphorylated Hesperidin (added before and after adsorption) | 500 $\mu$g/ml | | | | | |
| | 250 $\mu$g/ml | totally blocked | | | | |
| | 125 $\mu$g/ml | No plaques | | | | |
| | 62 $\mu$g/ml | | | | | |
| | 31 $\mu$g/ml | 27 | 20 | 24 | 24 | 9.9% |

Second trial results:

| Compound | Concentration | No. of Plaques | | | Average | % |
|---|---|---|---|---|---|---|
| Virus Control | – | 170 | 175 | 165<br>170 | 170 | 100% |
| Phosphorylated Hesperidin (during and after adsorption) | 500 µg/ml | None | | | | |
| | 250 µg/ml | None | | | | |
| | 125 µg/ml | 40 (estimate) | | | | |
| | 62 µg/ml | 35 | | 40 | 37.5 | 22% |
| | 31 µg/ml | 35 | | 40 | 32.5 | 17% |
| Phosphorylated Hesperidin (After adsorption only) | 500 µg/ml | None | | | | |
| | 250 µg/ml | None | | | | |
| | 125 µg/ml | As virus control | | | | |
| | 62 µg/ml | As virus control | | | | |
| | 31 µg/m | As virus control | | | | |

Third trial results:

| Compound | Concentration | No. of Plaques | Average | % |
|---|---|---|---|---|
| Virus Control | - | 190 188 195<br>189 185 192 | 189.8 | 100% |
| Phosphorylated Hesperidin (during adsorption only) | 3.75 µg/ml | As v.c. | | 100% |
| | 7.5 µg/ml | As v.c. | 141 | 100% |
| | 15 µg/ml | 148 140 135 | | 74% |
| | 31µg/ml | 120 115 111<br>(much smaller plaques) | 115 | 61% |
| | 62 µg/ml | monolayer destroyed | | |
| Phosphorylated Hesperidin (After adsorption only) | 3.75 µg/ml | As v.c. | | 100% |
| | 7.5 µg/ml | As v.c | | 100% |
| | 15 µg/ml | 120 125 131<br>(much smaller plaques) | 125 | 66% |
| | 31µg/ml | 119 125 128 | 124 | 65% |
| | 62 µg/ml | 90 92 85<br>(smaller plaques) | 89 | 47% |
| Phosphorylated Hesperidin (During and After Adsorption) | 3.75 µg/ml | 128 125 121 | 125 | 66% |
| | 7.5 µg/ml | 119 115 103 | 122 | 64% |
| | 15 µg/ml | 81 70 76 | 76 | 40% |
| | 31µg/ml | 63 60 58 | 60 | 32% |
| | 62 µg/ml | 50 45 48 | 48 | 25% |

EXAMPLE 7

Anti-viral activity of Phosphorylated Hesperidin

Cell Line: Vero
Virus: Herpes type 2 strain HG52

First trial results:

| Compound | Concentration | No. of Plaques | | | Average | % |
|---|---|---|---|---|---|---|
| Virus Control | – | 221 | 263 | 219<br>202 | 226 | 100% |
| Phosphorylated Hesperidin (during adsorption and incubation) | 100 µg/ml | 95 | 62 | | 78.5 | 35% |
| | 50 µg/ml | 112 | 132 | | 122 | 54% |
| | 10 µg/ml | 253 | 243 | | 248 | 100% |
| Phosphorylated Hesperidin (during adsorption | 100 µg/ml | 187 | 144 | | 166 | 73% |
| | 50 µg/ml | 154 | 146 | | 150 | 66% |
| | 10 µg/ml | 211 | 231 | | 221 | 98% |
| Virus Adsorption at room temperature;<br>Incubation at 37°C | | | | | | |

Second trial results:

| Compound | Concentration | No. of Plaques | | | Average | % |
|---|---|---|---|---|---|---|
| Virus Control | None | 137 | 140 | 146 | 141 | 100% |
| Phosphorylated Hesperidin (during adsorption) | 500 µg/ml | 50 | 58 | | 54 | 38% |
| Phosphorylated Hesperidin (during adsorption and incubation) | 500 µg/ml | None | | | 0 | 0% |
| | 250 µg/ml | 11 | 10 | | 10.5 | 7.5% |
| | 125 µg/ml | 58 | 71 | | 64.5 | 46 |
| Phosphorylated Hesperidin (incubation only) | 500 µg/ml | None | | | 0 | 0 |
| | 250 µg/ml | 16 | 11 | | 13.5 | 9.6% |
| | 125 µg/ml | 50 | 61 | | 55.5 | 39% |

## Claims

**1.** Use of a phosphorylated flavanoid having the following formula

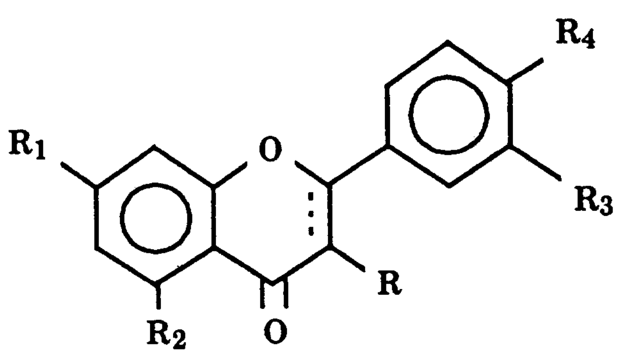

wherein R and $R_1$ are independently selected from hydrogen, hydroxy, methoxy, phosphonoxy and

saccharides linked via a hydroxy group selected from glucose, ribose, fructose, galactose, mannose and disaccharides composed of these saccharides;

$R_2$, $R_3$ and $R_4$ are independently selected from hydrogen, hydroxy, methoxy and phosphonoxy;
and wherein the dotted line indicates the optional presence of a double bond;
with the proviso that at least one of R, $R_1$, $R_2$, $R_3$ and $R_4$ is phosphonoxy;
or a pharmaceutically-acceptable acid addition salt thereof;

for the manufacture of a medicament for use in treating a retroviral, herpes simplex viral or cytomegaloviral infection.

**2.** Use according to claim 1, wherein the optional double bond is not present and R and $R_1$ to $R_4$ are each hydrogen, hydroxy, methoxy or phosphonoxy.

**3.** Use according to claim 1 or claim 2, wherein the optional double bond is not present and R is hydrogen.

**4.** Use according to any preceding claim, wherein the optional double bond is not present and $R_4$ is methoxy.

**5.** Use according to claim 3, wherein $R_4$ is methoxy and $R_1$ to $R_3$ are each hydroxy or phosphonoxy.

**6.** A phosphorylated flavanoid as defined in any of claims 1 to 5, for therapeutic use.

**7.** A method of treating a viral infection selected from the group consisting of a retroviral, herpes simplex viral or cytomegaloviral infection in a patient in need thereof which comprises administering to the patient an antivirally effective amount of a phosphorylated flavanoid as defined in any of claims 1 to 5.

European Patent Office

# PARTIAL EUROPEAN SEARCH REPORT

which under Rule 45 of the European Patent Convention shall be considered, for the purposes of subsequent proceedings, as the European search report

Application Number

EP 92 31 0277

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EUROPEAN JOURNAL OF BIOCHEMISTRY, vol. 190, no. 3, June 1990, pages 469-476; K. ONO et al.: "Differential inhibitory effects of various flavonoids on the activities of reverse transcriptase and cellular DNA and RNA polymerases" * Abstract; pages 473-475 * --- | 1-7 | A 61 K 31/665 |
| Y | SHOYAKUGAKU ZASSHI, vol. 43, no. 2, June 1989, pages 135-141, JP; T. KONOSHIMA et al.: "Studies on inhibitors of skin tumor promotion (V). Inhibitory effects of flavonoids on Epstein-Barr virus activation. II." * Abstract; page 136; page 138, table III * --- | 1-7 | |
| Y | EP-A-0 402 049 (K.K. HAYASHIBARA SEIBUTSU KAGAKU KENKYUJO) * Page 6, lines 4-6 * --- -/- | 1-7 | |

TECHNICAL FIELDS SEARCHED (Int. Cl.5)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

See sheet -C-

| Place of search | Date of completion of the search | Examiner |
|---|---|---|
| THE HAGUE | 01-03-1993 | KRAUTBAUER B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number

EP 92 31 0277

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| Y | DE-A-3 908 749 (L. GOERTZ)<br>* Whole document *<br>--- | 1-7 | |
| Y | QUINTESSENCE INTERNATIONAL, vol. 12, no. 2, February 1981, pages 133-138; G.T. TEREZHALMY et al.: "The treatment of herpetic infections: state of the art"<br>* Page 137 *<br>--- | 1-7 | |
| Y | DIE NATURWISSENSCHAFTEN, vol. 62, no. 6, June 1975, page 301; A. WACKER et al.: "Zur Virushemmung mit Hesperidin"<br>* Whole article *<br>--- | 1-7 | TECHNICAL FIELDS SEARCHED (Int. Cl.5) |
| Y | CONTRACEPTION, vol. 19, no. 1, January 1979, pages 95-106; C. JOYCE et al.: "Contraceptive effects of intravaginal application of acrosin and hyaluronidase inhibitors in rabbit"<br>* Abstract *<br>--- | 1-7 | |
| Y | PATENT ABSTRACTS OF JAPAN, vol. 12, no. 47 (C-475)(2894), 12th February 1988, page 153 C475; & JP-A-62 195 333 (KYODO KENKO SHIZEN SHOKUHIN K.K.) 28-08-1987<br>* Abstract *<br>----- | 1-7 | |

EP 92 31 0277 -C-

Remark : Although claim 7 is directed to a method of treatment of the human/animal body (Art. 52(4) EPC) the search has been carried out and based on the alleged effects of the compound/composition.